# EUROPEAN PATENT SPECIFICATION

(11) **EP 2 047 877 B1**
(45) Date of publication and mention of the grant of the patent: **28.12.2011**
(21) Application number: 07791197.2
(22) Date of filing: 23.07.2007
(51) Int. Cl.: A61M 5/315, A61M 5/20

(54) **CONSTANT VOLUME DISCHARGE DEVICE**
ABGABEVORRICHTUNG MIT KONSTANTEM VOLUMEN
DISPOSITIF DE DÉCHARGE DE VOLUME CONSTANT

(30) Priority: 28.07.2006 JP 2006205717
(43) Date of publication of application: 15.04.2009
(73) Proprietor: Taisei Kako Co., Ltd., Kita-ku Osaka-shi Osaka 531-0072 (JP); ZERIA PHARMACEUTICAL CO., LTD., Tokyo 103-8351 (JP); WU, Men-dar, Nagoya-shi, Aichi 465-0032 (JP)
(72) Inventor: WU, Men-dar, Nagoya-shi, Aichi 465-0032 (JP); OGAWA, Yukihiro, Ibaraki-shi, Osaka 567-0054 (JP)
(74) Representative: Dokter, Eric-Michael
(86) International application number: PCT/JP2007/064465
(87) International publication number: WO 2008/013149

(56) References cited:
- WO-A1-2005/009515
- JP-A- 2002 503 116
- JP-A- 2002 506 379
- JP-A- 2002 508 225
- JP-A- 2007 514 487
- US-A1- 2005 154 351
- US-A1- 2005 177 115

## Description

### Technical Field

The present invention relates to a constant-volume dispenser, and more specifically to a constant-volume dispenser for injection of a content from a nozzle hole by a predetermined amount through a movement of a syringe's plug in an axial direction of the syringe's container toward the nozzle hole.

### Background Art

In general, there is known a constant-volume dispensing syringe as disclosed in Patent Document 1 for example, which includes a pre-filled syringe (hereinafter simply called syringe) that is already filled with a medicinal liquid, and a constant-volume dispenser that moves the syringe's plug thereby injecting the medicinal agent from the syringe's container by a predetermined amount. In a constant-volume dispenser which is used in the constant-volume dispensing syringe, an operation member is moved whereby an inner cylinder housed in an outer cylinder is moved together with a pusher toward a nozzle hole, so that the pusher pushes the plug toward the nozzle hole. This moves the plug toward the nozzle hole, and the medicinal agent is injected from the nozzle hole.

Normally, the nozzle hole of the syringe's container is in a sealed state before the syringe is connected with the constant-volume dispenser. Therefore, if the pusher pushes the plug at the time when the syringe is connected with the constant-volume dispenser, there is a risk that the container will be ruptured. In the process of placing the plug, a variable amount of air is included in the container. Also, in the process of heat sterilization, the air expands and moves the plug by a variable amount. For these reasons, the position of the plug in the container is different from one syringe to another. In order to prevent container rupture even if there is a variation in the position of the plug from one syringe to another, the pusher in the constant-volume dispenser is positioned so as to ensure that a gap is provided between the pusher and the plug under the state where the syringe and the constant-volume dispenser are connected with each other.

Normally, such a constant-volume dispensing syringe as described above is used after a preparation operation in which the air in the container is discharged by moving the operation member, with the nozzle hole pointed upward. In other words, air is removed in the preparation operation prior to use. Conventionally, the air removal requires a plurality of preparation operations if the pusher's travel distance in a single preparation operation is shorter than needed due to the above-described gap provided between the pusher and the plug.
Patent Document 1: JP-A 9-503150

US 2005/0177115 A1 shows an injection device including a housing, a reservoir for a product to be injected and an advancing apparatus that carries out an advancing movement to discharge a selected dose of product from the reservoir, wherein the discharged dose is selected by the dosing movement.

### Disclosure of the Invention

### Problems to be Solved by the Invention

However, with the conventional constant-volume dispensing syringe, in a case where the user does not know how many preparation operations will be necessary in order to complete the air removal, careful operation must be made on the operation member in order not to waste the content, so there has been a problem that air removal takes time.

Therefore, a primary object of the present invention is to provide a constant-volume dispenser which allows easy and reliable air removal from the syringe.

### Means for Solving the Problems

According to an aspect of the present invention, there is provided a constant-volume dispenser connected with a syringe. The syringe includes a cylindrical container having an end with a nozzle hole; and a plug provided inside the container. The plug is moved axially of the container toward the nozzle hole for injection of a content contained in the container from the nozzle hole by a predetermined amount. The dispenser includes: an outer cylinder extending in the axial direction; an inner cylinder housed in the outer cylinder movably in the axial direction; a pusher inserted through the outer cylinder and the inner cylinder for movement with the inner cylinder in the axial direction toward the nozzle hole to push the plug; an operation member movable in the axial direction for movement in the axial direction toward the nozzle hole to move the inner cylinder and the pusher in the axial direction toward the nozzle hole; a stopping part for stopping the operation member at a predetermined stopping position; disposition means for disposing the operation member at an initial position which is further away from the nozzle hole than the stopping position in the axial direction; and returning means comprising an elastic member for returning the operation member from the stopping position to a stand-by position which is between the stopping position and the initial position. In a preparation operation the pusher is moved by a movement of the operation member from the initial position, at which the operation member is disposed by the disposition means, to the stopping position. After the preparation operation the pusher is moved by a movement of the operation member from the stand-by position, to which the operation member is returned by the elastic member of the returning means, to the stopping position. The pusher is moved by a shorter distance than in the preparation operation.

According to the present invention, the pusher's travel distance in the preparation operation is made longer whereby the plug's travel distance is made longer, which makes it possible to remove air from the syringe easily and reliably in a single preparation operation. Also, the longer travel distance of the pusher in the preparation operation allows to move the plug reliably even in a case where the plug is stuck in the container, and ensures smooth movement of the plug after the preparation operation.

Preferably, the stopping Part is provided in the outer cylinder. In cases where the operation member is provided in the outer cylinder or near the outer cylinder, providing the stopping part in the outer cylinder as disclosed in the above allows easy and reliable stoppage of the operation member.

Further preferably, the stopping part is provided in the inner cylinder which is stopped by the lid member. In this case, the inner cylinder which is moved by the operation member in the axial direction toward the nozzle hole is stopped by the lid member, and the stopping part in the inner cylinder stops the operation member. Providing the stopping part in the inner cylinder which is moved by the operation member as disclosed in the above allows easy and reliable stoppage of the operation member.

Further, preferably, the constant-volume dispenser further includes restriction means for restricting movement of the operation member disposed at the initial position by the disposition means so that the operation member will not move in the axial direction toward the nozzle hole. In this case, it becomes possible to prevent unintended movement of the operation member before use, and thus to prevent rupture of the syringe's container. This makes it possible to prevent leakage of the content from the syringe.

The above-described object, other objects, characteristics, aspects and advantages of the present invention will become more apparent from the following detailed description of embodiments to be made with reference to the attached drawings.

### Brief Description of the Drawings

Fig. 1 is a perspective view showing an embodiment of the present invention.
Fig. 2 is an exploded perspective view of the embodiment in Fig. 1.
Fig. 3 is a sectional view taken in lines X-X in the embodiment shown in Fig. 1.
Fig. 4 is a sectional view taken in lines Y-Y in the embodiment shown in Fig. 1.
Fig. 5 is a sectional view of an inner cylinder.
Fig. 6 is a sectional view of a lid member.
Fig. 7 is a sectional view showing a state where an operation member has been rotated from a state shown in Fig. 4.
Fig. 8 is a sectional view showing a preparation operation in the embodiment in Fig. 1.
Fig. 9 is an exploded perspective view of another embodiment of the present invention.
Fig. 10 is a sectional view of the embodiment in Fig. 9.
Fig. 11 is a sectional view showing a preparation operation in the embodiment in Fig. 9.

### Legend

- 10, 10a: Constant-volume dispensing syringes
- 12: Syringe
- 14, 14a: Constant-volume dispensers
- 16: Container
- 18: Plug
- 20a: Nozzle hole
- 26: Outer cylinder
- 28, 28a: Inner cylinders
- 30: Spring
- 32, 32a: Lid members
- 34, 34a: Pushers
- 36, 36a: Operation members
- 38b, 52a: Stopping parts
- 44, 46: Annular projections
- 48a, 48b, 48c, 48d, 58, 66, 74, 78, 84, 86, 86a, 92: Projections
- 50: Cutout
- 80, 94, 96: Cylindrical portions
- 98: Stopper
- Medicinal agent: L

### Best Mode for Carrying Out the Invention

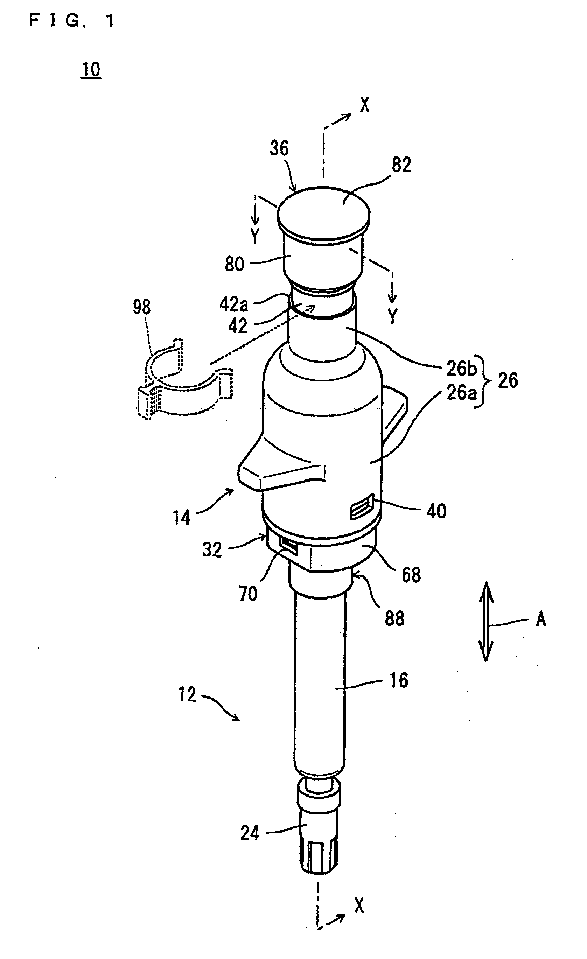

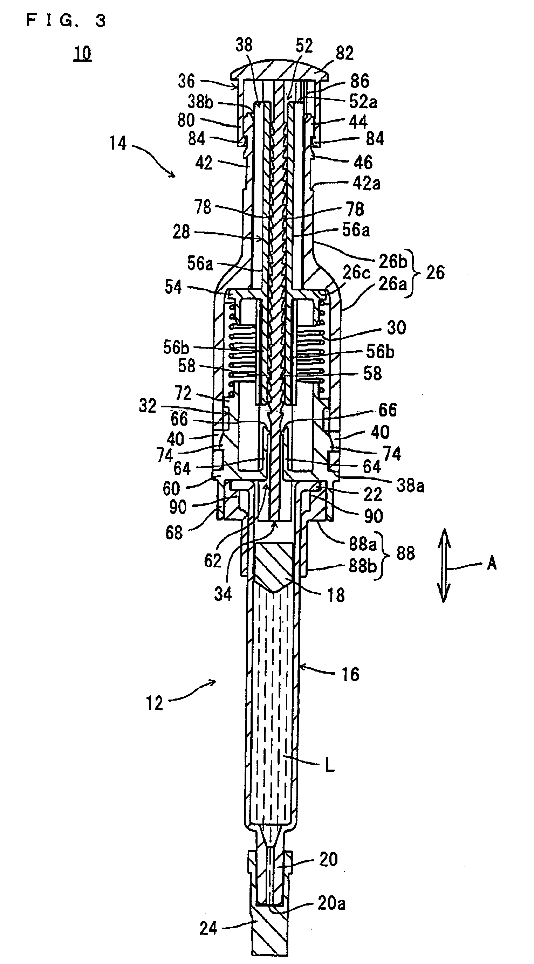

Hereinafter, description will be made for embodiments of the present invention, with reference to the drawings.
Referring to Fig. 1, a constant-volume dispensing syringe 10 as an embodiment of the present invention includes a pre-filled syringe (hereinafter will simply be called syringe) 12 which is pre-filled with a medicinal liquid L (see Fig. 3), and a constant-volume dispenser 14 for injection of the medicinal agent L as a content by a predetermined amount (50 µL for example) from the syringe 12.

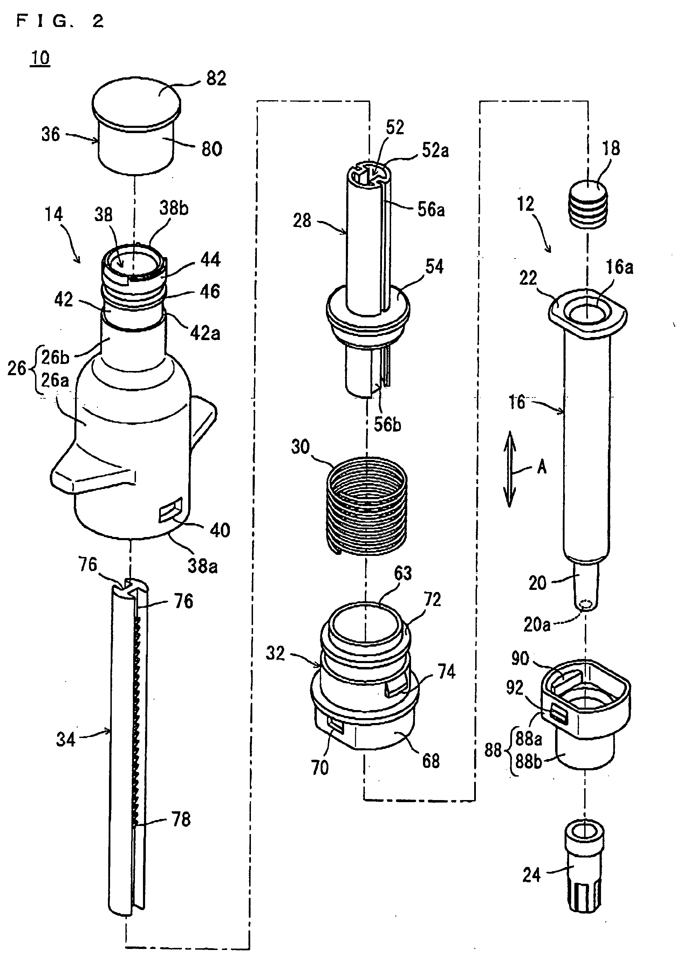

As shown in Fig. 2 and Fig. 3, the syringe 12 includes a cylindrical container 16 which extends in Arrow A direction (axial direction), and a plug 18 which is provided slidably within the container 16. The container 16 has an end provided with a nozzle 20. The nozzle 20 has a nozzle hole 20a for injecting the medicinal agent L. Also, the container 16 has another end provided with an insertion hole 16a into which a pusher 34 (to be described later) of the constant-volume dispenser 14 is inserted; and a flange 22. The container 16 as described is made of glass for example. A cap 24 is attached to the nozzle 20 of the container 16 to close the nozzle hole 20a.

The plug 18 is an elastic member made of butyl rubber for example. The plug 18 is formed into a generally columnar shape having a plurality of annular projections around its outer circumferential surface. Each annular projection of the plug 18 has an outer diameter which is greater than an inner diameter of the container 16 within a range which allows the plug 18 to slide inside the container 16. Since each annular projection of the plug 18 makes tight fit to the inner circumferential surface of the container 16, the medicinal agent L is prevented from leaking inside the container 16, i.e., from seeping around the plug 18 toward the side closer to the insertion hole 16a. The plug 18 as described above is disposed inside the container 16 by using such a method as vacuum plugging, air needle plugging and vent tube plugging, in order to minimize the volume of air remaining between the plug 18 and the nozzle hole 20a in the container 16 which holds the medicinal agent L.

As shown in Fig. 2 and Fig. 3, the constant-volume dispenser 14 includes an outer cylinder 26 which extends in the Arrow A direction; an inner cylinder 28 which is housed in the outer cylinder 26; a spring 30 which is housed in the outer cylinder 26; a lid member 32 which is fitted to the outer cylinder 26; a pusher 34 which penetrates the outer cylinder 26, the inner cylinder 28 and the lid member 32; and an operation member 36 which is attached to the outer cylinder 26.

The outer cylinder 26 is formed into a generally cylindrical shape which extends in the Arrow A direction, and has a through-hole 38. The outer cylinder 26 is composed of a large-diameter portion 26a which has an open end 38a, and a small-diameter portion 26b which has an open end 38b. As will be described later, the open end 38b of the outer cylinder 26 serves as a stopping part which stops the operation member 36 in the present embodiment. Hereinafter, the open end 38b will be called stopping part 38b.

As shown in Fig. 3, the small-diameter portion 26b which is closer to the operation member 36 is smaller in its outer and inner diameters than the large-diameter portion 26a which is closer to the syringe 12. Based on this, a step 26c is provided inside the outer cylinder 26. The outer cylinder 26 as described above is made of a synthetic resin such as PP (polypropylene).

The large-diameter portion 26a has an outer circumferential surface provided with two projections for a user to place his/her fingers. Also, as shown in Fig. 3, the large-diameter portion 26a has a side wall provided with two through-holes 40 arranged inline perpendicularly to the Arrow A direction.

The small-diameter portion 26b is provided with a disposition portion 42 which ranges from the stopping part 38b to near an intermediate region for disposition of the operation member 36. The disposition portion 42 includes annular projections 44, 46. Except for the annular projections 44, 46, the disposition portion 42 has an outer diameter which is smaller than that of the small-diameter portion 26b excluding the disposition portion 42. The annular projection 44 is provided near the stopping part 38b. The annular projection 46 is provided between the annular projection 44 and a step 42a of the disposition portion 42.

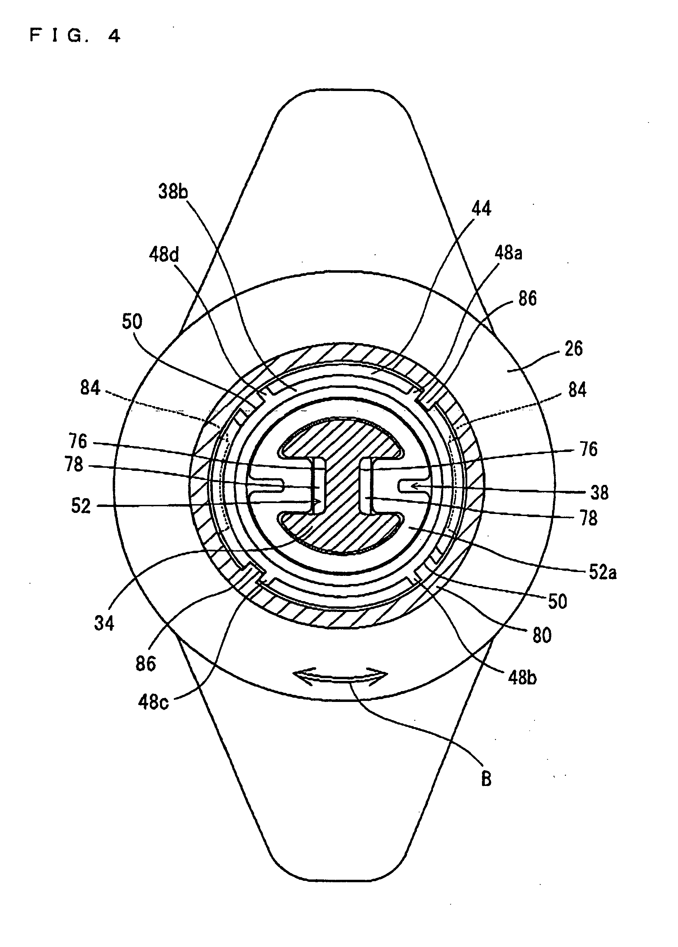

As shown in Fig. 4, the annular projection 44 is provided with a pair of projections 48a, 48b and a pair of projections 48c, 48d extending toward the stopping part 38b. Also, the annular projection 44 is provided with two cutouts 50 which extend perpendicularly to an Arrow B direction (circumferential direction) (i.e. extending in the Arrow A direction: see Fig. 2 and Fig. 3). In other words, the two cutouts 50 split the annular projection 44. One of the cutouts 50 is between the two projections 48a, 48b and near the projection 48b. The other of the cutouts 50 is between the two projections 48c, 48d and near the projection 48d. As shown in Fig. 3, the annular projection 46 has an outer diameter which becomes smaller toward the annular projection 44.

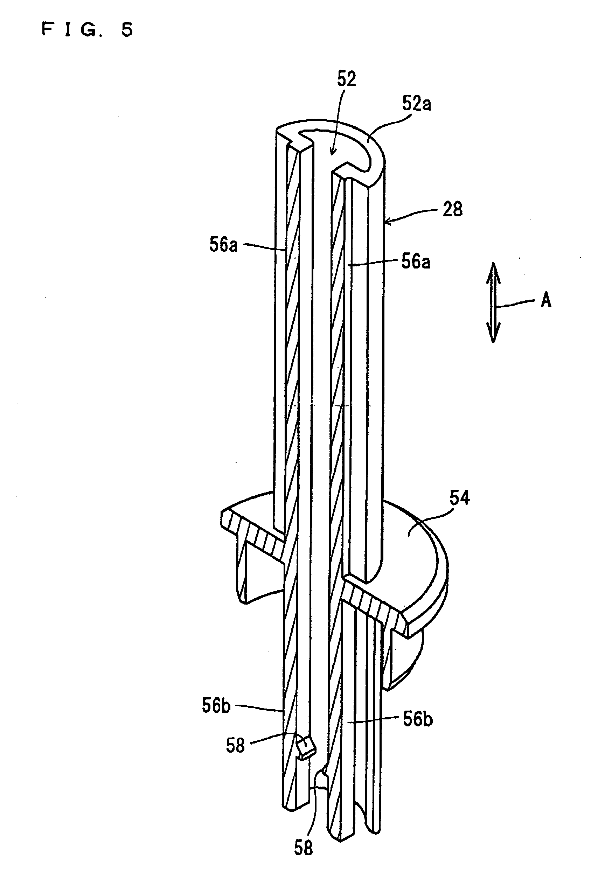

As shown in Fig. 2 and Fig. 5, the inner cylinder 28 is formed in a generally cylindrical shape which extends in the Arrow A direction, and has a through-hole 52. The inner cylinder 28 has an outer circumferential surface provided with a flange 54 at a position slightly closer to the syringe 12 than the intermediate position. As shown in Fig. 5, the inner cylinder 28 has a side wall provided with a pair of inward recesses 56a extending from an open end 52a to the flange 54. On the side wall of the inner cylinder 28, the recesses 56a are arranged inline in a perpendicular direction to the Arrow A direction. Due to this arrangement, the through-hole 52 of the inner cylinder 28 has a generally I-shaped look in a front view as taken on the open end 52a (see Fig. 4). Also, the side wall of the inner cylinder 28 is provided with a pair of inward recesses 56b extending from the flange 54 toward the syringe 12. The recesses 56b are each separated from the other parts of the side wall of the inner cylinder 28. Also, each of the recesses 56b is inline with one of the recesses 56a in the Arrow A direction. The pair of recesses 56b have mutually opposing surfaces, each of which is provided with a saw-tooth like projection 58 slanted to become high toward the syringe 12. The inner cylinder 28 as described above is made of a synthetic resin such as ABS (Acrylonitrile Butadiene Styrene).

As shown in Fig. 2, the spring 30 is provided by a coil spring which can be obtained by winding a bar-like member into a coil. The spring 30 is made of a metal such as stainless steel.

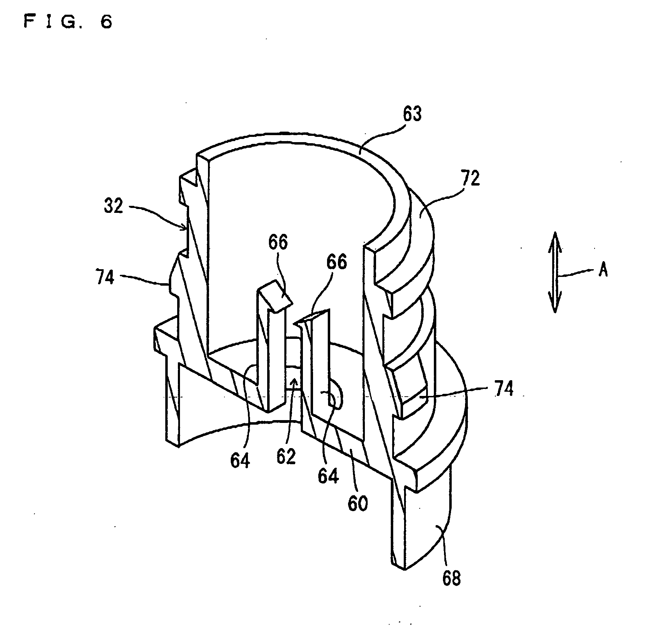

As shown in Fig. 6, the lid member 32 is formed generally in a cylindrical shape having a bottom plate 60. The bottom plate 60 has a center through-hole 62 which has generally the same shape as the through-hole 52 in the inner cylinder 28. The bottom plate 60 has a surface (upper surface as in Fig. 6) which is closer to an open end 63, and on this surface, there is erected a pair of click arms 64 arranged inline with the pair of recesses 56b (see Fig. 3) of the inner cylinder 28 in the Arrow A direction. As shown in Fig. 3 and Fig. 6, the click arms 64 extend toward the inner cylinder 28, with their ends forming a mutually pointing pair of saw-tooth like projections 66 which become higher toward the syringe 12.

Also, the bottom plate 60 has a surface (lower surface as in Fig. 6) which faces the syringe 12, and this surface is provided with a fitting portion 68 into which a connecting member 88 (to be described later) is fitted. As shown in Fig. 2, the fitting portion 68 has through-holes 70. It should be noted here that although Fig. 2 shows only one through-hole 70, two through-holes 70 are made in the fitting portion 68, in line in a direction perpendicularly to the Arrow A direction.

A flange 72 is provided near the open end 63, around the outer circumferential surface of the lid member 32. On the outer circumferential surface of the lid member 32 and more closely to the syringe 12 than is the flange 72, projections 74 are provided to fit into the through-holes 40 of the outer cylinder 26. The lid member 32 as described above is made of a synthetic resin such as PP.

As shown in Fig. 2 through Fig. 4, the pusher 34 is formed into a generally columnar shape to correspond to the through-hole 52 of the inner cylinder 28. Specifically, the pusher 34 is formed into a generally columnar shape which has two recesses 76 recessing from two opposing directions which are perpendicular to the Arrow A direction. The pusher 34 has a generally I-shaped section across the direction perpendicular to the Arrow A direction (see Fig. 4). As shown in Fig. 3, the two recesses 76 each have a bottom surface provided with a plurality of equi-distanced projections 78. Each projection 78 is formed like a saw tooth which becomes lower toward the syringe 12. The pusher 34 as described above is made of a synthetic resin such as POM (polyacetal).

As shown in Fig. 2 and Fig. 3, the operation member 36 is formed in a cylindrical shape opening toward the syringe 12. The operation member 36 includes a cylindrical portion 80 which extends in the Arrow A direction, and a disc-like button 82 provided on the cylindrical portion 80. As shown in Fig. 3 and Fig. 4, two arc-shaped projections 84 are provided in an opposed manner on an inner circumferential surface of the cylindrical portion 80, near the opening. The projections 84 are between the annular projections 44, 46 of the disposition portion 42 until use is made. In the present embodiment, the annular projections 44, 46 of the disposition portion 42 and the two projections 84 of the operation member 36 constitute disposition means. The position shown in Fig. 3 is an initial position of the operation member 36.

Also, as shown in Fig. 3, the inner circumferential surface of the cylindrical portion 80 is provided with projections 86 which extend in the Arrow A direction. As shown in Fig. 4, one of the projections 86 is disposed between the projections 48a, 48b of the outer cylinder 26 while the other is disposed between the projections 48c, 48d of the outer cylinder 26. It should be noted here that Fig. 3 shows only one of the projections 86. Under the state shown in Fig. 3 and Fig. 4, the two projections 86 make contact with the annular projection 44, i.e. contact with a surface (upper surface in Fig. 3) which is closer to the stopping part 38b. Therefore, under the state shown in Fig. 3 and Fig. 4, pushing the button 82 in the Arrow A direction toward the syringe 12 does not move the operation member 36. In the present embodiment, the annular projection 44 of the disposition portion 42 and the two projections 86 of the operation member 36 constitute restriction means.

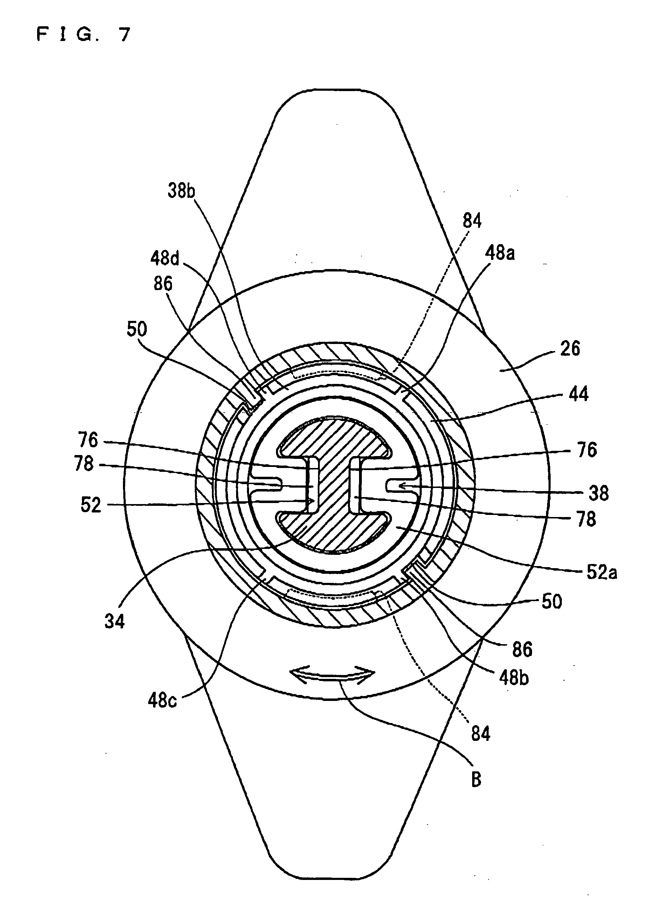

When operating the operation member 36, the operation member 36 is turned as shown in Fig. 7, in one of the directions indicated by Arrow B (in a clockwise direction in the present description), from the state shown in Fig. 4, so that the two projections 86 are positioned above their respective cutouts 50. Then, under this state, the button 82 is pressed to move the operation member 36 in the Arrow A direction toward the syringe 12. The operation member 36 which is being moved in the Arrow A direction toward the syringe 12 is stopped when the button 82 makes contact with the stopping part 38b of the outer cylinder 26 within the cylindrical portion 80. In other words, the operation member 36 is stopped by the stopping part 38b of the outer cylinder 26.

The constant-volume dispenser 14 is obtained by assembling the above-described members in the following procedure for example: First, the pusher 34 is inserted into the inner cylinder 28 so that each projection 58 will be between two consecutive ones of the projections 78 arranged inline in the Arrow A direction. Subsequently, the inner cylinder 28 into which the pusher 34 has been inserted, and the spring 30 are placed into the outer cylinder 26 from the open end 38a; the inner cylinder 28 is inserted first and then the spring 30 follows. Then, the lid member 32 is fitted around the outer cylinder 26 from the open end 38a. The lid member 32 is fixed to the outer cylinder 26 when the projections 74 are fitted into the through-holes 40. Thereafter, as the projection 84 of the operation member 36 rises and passes over the annular projection 44 of the disposition portion 42, the assembly of the operation member 36 to the outer cylinder 26 is complete.

As shown in Fig. 3, according to the constant-volume dispenser 14, the spring 30 is pressed between the flange 54 of the inner cylinder 28 and the flange 72 of the lid member 32, whereby the inner cylinder 28 is urged in the Arrow A direction away from the syringe 12, causing the flange 54 of the inner cylinder 28 to make contact with the step 26c of the outer cylinder 26. Under the state where the flange 54 makes contact with the step 26c, the open end 52a of the inner cylinder 28 protrudes from the stopping part 38b of the outer cylinder 26. Also, until use is made, the pusher 34 which is inserted through the inner cylinder 28 makes contact with the button 82 of the operation member 36. In the present embodiment, the inner cylinder 28, the spring 30 and the lid member 32 constitute returning means.

Also, as shown in Fig. 2 and Fig. 3, the constant-volume dispensing syringe 10 includes the connecting member 88 for connecting the syringe 12 with the constant-volume dispenser 14. The connecting member 88 is constituted by a large-diameter portion 88a which has a cylindrical shape and is generally oval in its outer shape, and a small-diameter portion 88b which has a cylindrical shape and has an inner diameter slightly greater than the outer diameter of the container 16. As shown in Fig. 2 and Fig. 3, the large-diameter portion 88a has an inner circumferential surface provided with two projections 90. Also, as shown in Fig. 2, the large-diameter portion 88a has an outer circumferential surface provided with projections 92 to fit into the through-holes 70 of the lid member 32. It should be noted here that although Fig. 2 shows only one of the projections 92, two projections 92 are provided inline in a direction perpendicular to the Arrow A direction on the outer circumferential surface of the large-diameter portion 88a.

The syringe 12 and the constant-volume dispenser 14 are connected with each other when the container 16 is inserted into the connecting member 88 and the large-diameter portion 88a of the connecting member 88 is inserted into the fitting portion 68 of the lid member 32. The connecting member 88 is fixed to the lid member 32 as the projections 92 of the large-diameter portion 88a is fitted into the through-holes 70 of the fitting portion 68. As shown in Fig. 3, under the state where the connecting member 88 is fixed to the lid member 32, the flange 22 of the container 16 is clamped between the bottom plate 60 of the lid member 32 and the projections 90 of the large-diameter portion 88a. Also, the inner circumferential surface of the small-diameter portion 88b makes contact with the outer circumferential surface of the container 16, and the inner circumferential surface of the large-diameter portion 88a makes contact with the outer circumferential surface of the flange 22. Therefore, the syringe 12 which is connected with the constant-volume dispenser 14 does not move in directions perpendicular to the Arrow A direction nor in the Arrow A direction. Also, it should be understood from Fig. 3 that the pusher 34 of the constant-volume dispenser 14 is disposed so as to provide a space between the pusher 34 and the plug 18 until use is made.

Normally, using a vacuum plugging, air-needle plugging or vent tube plugging method, etc. in order to dispose the plug 18 inside the container 16 will unavoidably allow air to remain inside the container 16 if the container 16 already has the cap 24 attached and holds the medicinal agent L. For this reason, a preparation operation for discharging the air remaining in the container 16 is performed before use is made of the constant-volume dispensing syringe 10. In other words, the constant-volume dispensing syringe 10 is used after the air is removed by the preparation operation.

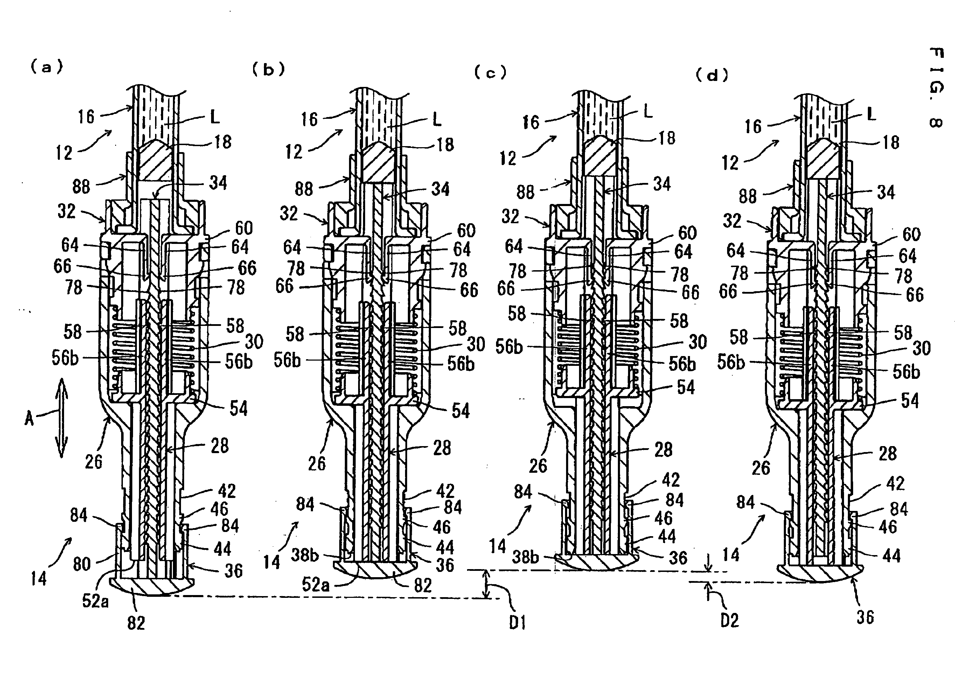

Next, an operation of the constant-volume dispensing syringe 10 at the time of preparation operation will be described with reference to Fig. 8. The preparation operation is performed under a state where an unillustrated injection needle is attached to the nozzle 20 (see Fig. 3) in place of the cap 24. Also, as shown in Fig. 8(a), the preparation operation is performed with the button 82 being pointed downward. In other words, the preparation operation is performed with the nozzle hole 20a (see Fig. 3) being pointed upward thereby letting the air gather on the side formed with the nozzle hole 20a.

First, the operation member 36 which is at its initial position {see Fig. 8 (a)} is pressed. In this movement, the cylindrical portion 80 makes elastic deformation to cause the projection 84 to ride and pass over the annular projection 46 of the disposition portion 42, allowing the operation member 36 to move in the Arrow A direction toward the syringe 12. This causes the pusher 34 to be pressed by the operation member 36 to move in the Arrow A direction toward the syringe 12.

Subsequently, the first set of the projections 78 in the pusher 34 makes contact with the projections 66 in the click arms 64. Since the projections 66 have a saw-tooth shape which becomes higher toward the syringe 12, and the projections 78 have a saw-tooth shape which becomes lower toward the syringe 12, top portions of the projections 66 slip on the slopes of the projections 78 as the pusher 34 moves, and the projections 66 are pressed outward by the projections as the pusher 34 moves. This causes elastic deformation in the pair of click arms 64 near the bottom plate 60, making the click arms 64 swing so that their two projections 66 go away from each other.

Then, as shown in Fig. 8(b), the projection 84 of the operation member 36 rides and passes over the annular projection 46 of the disposition portion 42, whereby the first set of projections 78 of the pusher 34 rides and passes over the projections 66 of the click arms 64 inside the outer cylinder 26. Meanwhile, in the operation member 36, the open end 52a of the inner cylinder 28 makes contact with the button 82. At least by the time the open end 52a of the inner cylinder 28 makes contact with the button 82, the pusher 34 makes contact with the plug 18. In other words, the plug 18 is disposed inside the container 16 so that the plug 18 makes contact with the pusher 34 at least under the state shown in Fig. 8(b) even if there is a variation in the position of the plug 18 from one syringe 12 to another resulting from manufacturing processes.

From the state shown in Fig. 8 (b), the operation member 36 is pressed against the urge from the spring 30. This moves the inner cylinder 28 and the pusher 34 in the Arrow A direction toward the syringe 12. In association with this, the plug 18 which is pressed by the pusher 34 moves in the Arrow A direction toward the nozzle hole 20a (see Fig. 3), to discharge the air in the container 16 through the injection needle attached to the nozzle 20 (see Fig. 3) to the outside.

Then, as shown in Fig. 8(c), the operation member 36 stops as it makes contact with the stopping part 38b of the outer cylinder 26. In other words, the position shown in Fig. 8 (c) is the stopping position of the operation member 36. By moving the operation member 36 in this way from the initial position {see Fig. 8 (a)} to the stopping position {see Fig. 8(c)}, the pusher 34 is moved to the injection-starting position (the position where the second projection 78 rides and passes over the projection 66 in the present description). This moves the plug 18 to the position where the air removal is complete.

Thereafter, as the pressure is removed from the operation member 36, the urge from the spring 30 moves the inner cylinder 28 and the operation member 36 in the Arrow A direction away from the syringe 12. In this movement, the pusher 34 does not move together with the inner cylinder 28 in the Arrow A direction away from the syringe 12 because the projections 78 of the pusher 34 and the projections 66 of the lid member 32 engage with each other on their mutually parallel faces. Also, since the projections 58 are formed like saw teeth each becoming higher toward the syringe 12 and the projections 78 are formed like saw teeth each becoming lower toward the syringe 12, tops of the projections 58 slip on the slanted surfaces of the projections 78 as the inner cylinder 28 moves in the Arrow A direction away from the syringe 12. Then, as the inner cylinder 28 continues to move in the Arrow A direction away from the syringe 12, the projections 58 are pressed outward by the projections 78. This causes elastic deformation of the pair of recesses 56b near the flange 54, making the recesses 56b swing so that their two projections 58 go away from each other. Thereafter, as shown in Fig. 8 (d), when the projections 58 ride and pass over one set of the projections 78 and the projection 84 makes contact with the annular projection 46, the operation member 36 is stopped. In other words, the position shown in Fig. 8(d) is a stand-by position of the operation member 36.

After such a preparation operation as described above, the operation member 36 is moved from the stand-by position {see Fig. 8(d)} to the stopping position {see Fig. 8(c)}, whereby the projections 58 of the inner cylinder 28 and the projections 78 of the pusher 34 engage with each other on their mutually parallel faces, and the pusher 34 moves with the inner cylinder 28 in the Arrow A direction toward the syringe 12. This causes the pusher 34 to move the plug 18 in the Arrow A direction toward the nozzle hole 20a (see Fig. 3) by a predetermined distance.

As understood from Fig. 8, in the constant-volume dispenser 14, a travel distance D1 of the operation member 36 in the preparation operation is longer than a travel distance D2 of the operation member 36 after the preparation operation, and a travel distance of the pusher 34 in the preparation operation is longer than a travel distance of the pusher 34 after the preparation operation. According to this arrangement, it is possible to make a travel distance of the plug 18 in the preparation operation longer than a travel distance of the plug 18 after the preparation operation.

According to the constant-volume dispensing syringe 10 as described above, it is possible to arrange that a single preparation operation ensures to move the plug 18 to a position where complete air removal has been made, by making the travel distance of the pusher 34 in the preparation operation longer than the travel distance of the pusher 34 after the preparation operation. Therefore, it is possible to remove air from the syringe 12 easily and reliably in a single preparation operation.

A long travel distance of the pusher 34 in the preparation operation allows to move the plug 18 reliably even in a case where the plug 18 is stuck in the container 16, and ensures smooth movement of the plug 18 after the preparation operation.

Providing the stopping part 38b in the outer cylinder 26 allows easy and reliable stoppage of the operation member 36 provided in the outer cylinder 26.

The contact between the projection 86 and the annular projection 44 when the operation member 36 is at the initial position prevents the operation member 36 from moving in the Arrow A direction toward the syringe 12. This makes it possible to prevent unintended movement of the operation member 36 before use is made, and to eliminate rupture of the container 16. This makes it possible to prevent leakage of the medicinal agent L from the syringe 12.

It should be noted here that in the constant-volume dispenser 14 described above, the stopping part 38b which stops the operation member 36 is provided by an open end of the outer cylinder 26 which faces the operation member 36. However, the stopping part may be provided at a different location in the outer cylinder. For example, the disposition portion 42 may be formed at a position which makes the step 42a closer to the operation member 36 than shown in Fig. 3, so that the step 42a will stop the operation member 36.

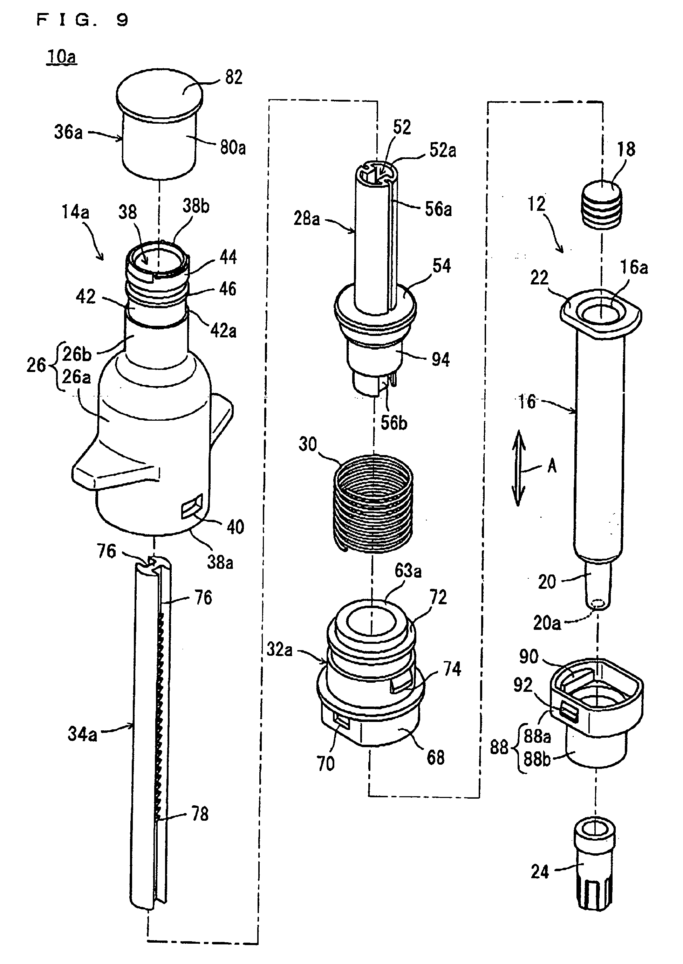

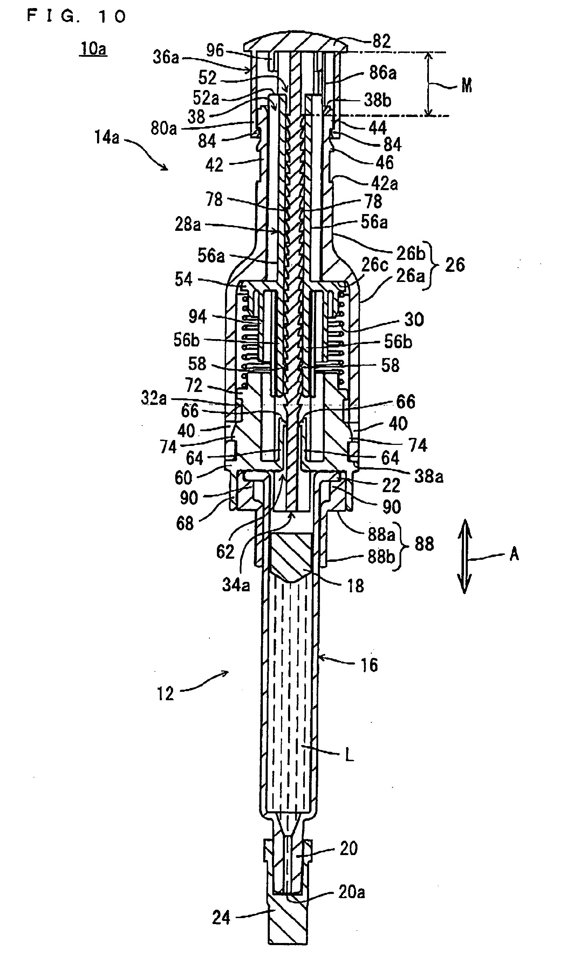

Next, another embodiment of the present invention will be described with reference to Fig. 9 and Fig. 10.
A constant-volume dispensing syringe 10a shown in Fig. 9 and Fig. 10 uses a constant-volume dispenser 14a in place of the constant-volume dispenser 14 described above. The constant-volume dispenser 14a uses an inner cylinder 28a, a lid member 32a, a pusher 34a and an operation member 36a in place of the inner cylinder 28, the lid member 32, the pusher 34 and the operation member 36. Otherwise, the constant-volume dispensers 14 and 14a are essentially the same, and therefore no repetitive description will be given. Also, the inner cylinder 28a, the lid member 32a, the pusher 34a and the operation member 36a include parts and portions which are essentially the same as those included in the inner cylinder 28, the lid member 32, the pusher 34 and the operation member 36. Such parts and portions will be indicated by the same reference symbols, and no repetitive description will be given.

As will be described later, in the present embodiment, the open end 52a of the inner cylinder 28 serves as the stopping part which stops the operation member 36a. Hereinafter, the open end 52a will be called the stopping part 52a.

In the inner cylinder 28a, a cylindrical portion 94 surrounding the pair of recesses 56b is formed on a surface of the flange 54 which is closer to the syringe 12. As is clear from comparison between Fig. 2 and Fig. 9, the lid member 32a has a thicker side wall than the lid member 32 so that an opening of the lid member 32a toward the operation member 36a has a smaller diameter than an opening of the lid member 32 toward the operation member 36. Therefore, an open end 63a of the lid member 32a has a greater surface area than the open end 63 of the lid member 32.

The operation member 36a has a cylindrical portion 80a which is formed to have a greater dimension in the Arrow A direction than does the cylindrical portion 80 of the operation member 36. Likewise, as shown in Fig. 10, the operation member 36a has a projection 86a which is formed to have a greater dimension in the Arrow A direction than does the projection 86 of the operation member 36. Also, as shown in Fig. 10, the button 82 is provided with a cylindrical portion 96 on a surface facing toward the syringe 12. The cylindrical portion 96 is provided so as to surround an outer circumferential surface of the pusher 34a when the constant-volume dispenser 14a is in the assembled state.

As shown in Fig. 10, the pusher 34a is extended in the Arrow A direction as compared with the pusher 34 so that a dimension (indicated by a symbol M in Fig. 10) from the projection 78 which is closest to the operation member 36a to the end which faces the surface of the operation member 36a is greater than in the pusher 34. Due to this arrangement, the pusher 34a makes contact with the button 82 of the operation member 36a under the state where the operation member 36a is disposed at its initial position.

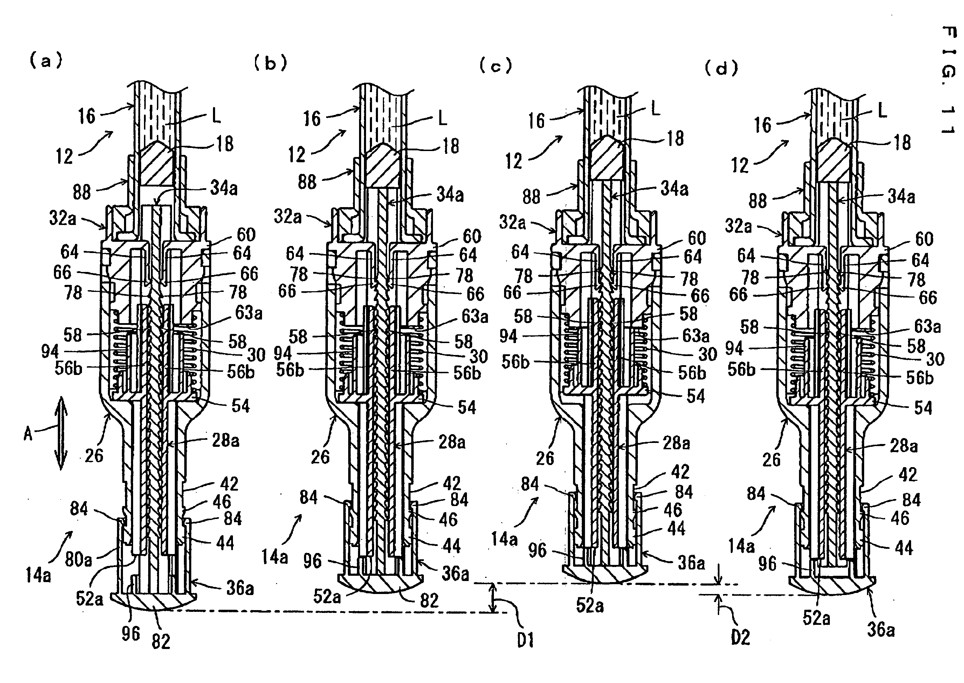

Next, with reference to Fig. 11, description will cover a process of a preparation operation in the constant-volume dispensing syringe 10a including the constant-volume dispenser 14a.
First, the operation member 36a which is at its initial position {see Fig. 11(a)} is pressed. In this movement, the pusher 34a is pressed by the operation member 36a and is moved in the Arrow A direction toward the syringe 12. Then, as shown in Fig. 11 (b), the projection 84 of the operation member 36a rides and passes over the annular projection 46 of the disposition portion 42, upon which the open end of the cylindrical portion 96 in the operation member 36a makes contact with the stopping part 52a of the inner cylinder 28a.

From the state shown in Fig. 11(b), the operation member 36a is pressed against the urge from the spring 30. This moves the inner cylinder 28a and the pusher 34a in the Arrow A direction toward the syringe 12. In association with this, the plug 18 which is pressed by the pusher 34a moves in the Arrow A direction toward the nozzle hole 20a (see Fig. 10), to discharge the air in the container 16 to the outside.

Then, as shown in Fig. 11 (c), the open end of the cylindrical portion 94 in the inner cylinder 28a makes contact with the open end 63a of the lid member 32a to stop the inner cylinder 28a, whereby the operation member 36a making contact with the stopping part 52a of the inner cylinder 28a stops. When the operation member 36a stops at its stopping position shown in Fig. 11 (c), the pusher 34a is moved to the injection-starting position, and the air removal is complete.

Thereafter, as the pressure is removed from the operation member 36a, the urge from the spring 30 moves the inner cylinder 28a and the operation member 36a in the Arrow A direction away from the syringe 12. In this movement, the operation member 36a is moved back to the stand-by position shown in Fig. 11 (d).

As understood from Fig. 11, in the constant-volume dispenser 14a, a travel distance D1 of the operation member 36a in the preparation operation is longer than a travel distance D2 of the operation member 36a after the preparation operation, and a travel distance of the pusher 34a in the preparation operation is longer than a travel distance of the pusher 34a after the preparation operation, just like in the constant-volume dispenser 14 described earlier. Therefore, according to the constant-volume dispensing syringe 10a, it is possible to remove air from the syringe 12 easily and reliably in a single preparation operation, just like in the constant-volume dispensing syringe 10 described earlier.

Providing the stopping part 52a in the inner cylinder 28a which is moved by the operation member 36a allows easy and reliable stoppage of the operation member 36a.

It should be noted here that in the constant-volume dispenser 14a described above, description was made for a case where the operation member 36a is stopped by the stopping part 52a which is the open end of the inner cylinder 28a that faces the operation member 36a. However, the stopping part may be provided at a different location in the inner cylinder. For example, a flange may be formed on the side wall of the inner cylinder 28a, closely to the open end which faces the operation member 36a, so that this flange will stop the operation member 36a.

It should be noted here that in the embodiments described above, description was made for a case where the restriction means is constituted by the annular projection 44 and the projection 86 (86a). However, the restriction means is not limited to this. For example, the restriction means may be provided by a stopper 98 which is drawn in broken lines in Fig. 1, and the stopper 98 may be attached between the step 42a of the disposition portion 42 and the open end of the cylindrical portion 80.

Also, in the above-described embodiments, description was made for a case where disposition means is constituted by the annular projections 44, 46 of the disposition portion 42 and two projections 84 of the operation member 36 (36a). However, the disposition means is not limited to this. For example, a connecting portion which is breakable by a pushing or rotating operation may be used to connect the outer cylinder with the operation member to dispose the operation member at its initial position.

Further, in the above-described embodiments, description was made for a case where a coil spring is used as the spring 30 to serve as the elastic member in constituting returning means. However, the elastic member included in the returning means is not limited to this. For example, the returning means may include a leaf spring, rubber, etc.

The present invention being thus far described and illustrated in detail, it is obvious that these description and drawings only represent examples of the present invention, and should not be interpreted as limiting the invention. The scope of the present invention is only limited by words used in the accompanied claims.

## Claims

1. A constant-volume dispenser (14) connected with a syringe (12) including a cylindrical container (16) having an end with a nozzle hole (20a) and a plug (18) provided inside the container (16), the plug (18) being moved axially of the container (16) toward the nozzle hole (20a) for injection of a content contained in the container (16) from the nozzle hole (20a) by a predetermined amount, the dispenser (14) comprising:
an outer cylinder (26) extending in the axial direction;
an inner cylinder (28) housed in the outer cylinder (26) movably in the axial direction;
a pusher (34) inserted through the outer cylinder (26) and the inner cylinder (28) for movement with the inner cylinder (28) in the axial direction toward the nozzle hole (20a) to push the plug (18);
disposition means (44, 46, 84) for disposing an operation member (36) at an initial position before a preparation operation for discharging air in the container (16);
the operation member (36) being arranged such that during the preparation operation a movement of the operation member (36) in the axial direction toward the nozzle hole (20a) causes the inner cylinder (28) and the pusher (34) to move in the axial direction toward the nozzle hole (20a);
a stopping part (38b) being arranged such that it stops the movement of the operation member (36) toward the nozzle hole (20a) at a predetermined stopping position; **characterised by**
returning means comprising an elastic member being arranged such that it urges the operation member (36) from the stopping position to a stand-by position which is between the stopping position and the initial position in axial direction;
wherein after the preparation operation during use of the constant-volume dispenser (14) the pusher (34) is moved by a movement of the operation member (36) from the stand-by position to the stopping position, the pusher (34) being moved by a shorter distance than in the preparation operation.

2. The constant-volume dispenser (14) according to Claim 1, wherein the stopping part (38b) is provided in the outer cylinder (26).

3. The constant-volume dispenser (14a) according to Claim 1, further comprising a lid member provided at an end on the nozzle-hole side of the outer cylinder (26) for stopping the inner cylinder (28a), wherein the stopping part (52b) is provided in the inner cylinder (28a) which is stopped by the lid member.

4. The constant-volume dispenser (14) according to one of Claims 1 through 3, further comprising restriction means for restricting movement of the operation member (36) disposed at the initial position by the disposition means (44, 46, 84) so that the operation member (36) will not move in the axial direction toward the nozzle hole (20a).

## Patentansprüche

1. Eine Konstantes-Volumen-Ausgabevorrichtung (14), die mit einer Spritze (12) verbunden ist, die einen ein Ende mit einem Düsenloch (20a) aufweisenden zylinderförmigen Behälter (16) und einen in dem Behälter (16) vorgesehenen Pfropfen (18) aufweist, wobei der Pfropfen (18) axial zu dem Behälter (16) in Richtung zu dem Düsenloch (20a) bewegt wird für ein Injizieren eines in dem Behälter (16) enthaltenen Inhalts in einer vorbestimmten Menge aus dem Düsenloch (20a), wobei die Ausgabevorrichtung (14) aufweist:
einen äußeren Zylinder (26), der sich in axialer Richtung erstreckt,
einen inneren Zylinder (28), der in dem äußeren Zylinder (26) in Axialrichtung bewegbar aufgenommen ist,
einen Drücker (34), der durch den äußeren Zylinder (26) und den inneren Zylinder (28) hindurch für ein Bewegen mit dem inneren Zylinder (28) in Axialrichtung in Richtung zu dem Düsenloch (20a) eingesetzt ist, um gegen den Pfropfen (18) zu drücken,
Anordnungsmittel (44, 46, 84) zum Anordnen eines Betätigungselements (36) in einer Anfangsposition vor einem Vorbereitungsvorgang zum Ablassen von Luft in dem Behälter (16),
wobei das Betätigungselement (36) derart angeordnet ist, dass während des Vorbereitungsvorgangs ein Bewegen des Betätigungselements (36) in Axialrichtung in Richtung zu dem Düsenloch (20a) bewirkt, dass sich der innere Zylinder (28) und der Drücker (34) in Axialrichtung in Richtung zu dem Düsenloch (20a) bewegen,
einen Stopperteil (38b), der derart angeordnet ist, dass er die Bewegung des Betätigungselements (36) in Richtung zu dem Düsenloch (20a) in einer vorbestimmten Stopp-Position anhält, **gekennzeichnet durch**:
ein Rückführmittel, aufweisend ein elastisches Element, das derart angeordnet ist, dass es das Betätigungselement (36) aus der Stopp-Position in eine Bereitschaftsposition zwingt, die in axialer Richtung zwischen der Stopp-Position und der Anfangsposition liegt,
wobei nach dem Vorbereitungsvorgang während der Verwendung der Konstantes-Volumen-Ausgabevorrichtung (14) der Drücker (34) **durch** eine Bewegung des Betätigungselements (36) aus der Bereitschaftsposition in die Stopp-Position bewegt wird, wobei der Drücker (34) um eine kürzere Entfernung bewegt wird als beim Vorbereitungsvorgang.

2. Die Konstantes-Volumen-Ausgabevorrichtung (14) gemäß Anspruch 1, wobei der Stopperteil (38b) in dem äußeren Zylinder (26) vorgesehen ist.

3. Die Konstantes-Volumen-Ausgabevorrichtung (14a) gemäß Anspruch 1, ferner ein Deckelelement aufweisend, das an einem Ende auf der Düsenlochseite des äußeren Zylinders (26) zum Anhalten des inneren Zylinders (28a) vorgesehen ist, wobei der Stopperteil (52b) in dem inneren Zylinder (28a) vorgesehen ist, der durch das Deckelelement angehalten wird.

4. Die Konstantes-Volumen-Ausgabevorrichtung (14) gemäß einem der Ansprüche 1 bis 3, ferner ein Beschränkungsmittel zum Beschränken der Bewegung des Betätigungselements (36) aufweisend, das mittels der Anordnungsmittel (44, 46, 84) in der Anfangsposition angeordnet ist, so dass das Betätigungsmittel (36) sich nicht in Axialrichtung in Richtung zu dem Düsenloch (20a) bewegt.

## Revendications

1. Distributeur à volume constant (14) raccordé avec une seringue (12) comprenant un récipient cylindrique (16) ayant une extrémité avec un trou de buse (20a) et un bouchon (18) prévu à l'intérieur du récipient (16), le bouchon (18) étant déplacé de manière axiale par rapport au récipient (16) vers le trou de buse (20a) pour l'injection d'un contenu, contenu dans le récipient (16) à partir du trou de buse (20a) selon une quantité prédéterminée, le distributeur (14) comprenant :
un cylindre externe (26) s'étendant dans la direction axiale ;
un cylindre interne (28) logé dans le cylindre externe (26) de manière mobile dans la direction axiale ;
un poussoir (34) inséré à travers le cylindre externe (26) et le cylindre interne (28) pour le mouvement avec le cylindre interne (28) dans la direction axiale vers le trou de buse (20a) afin de pousser le bouchon (18) ;
des moyens de disposition (44, 46, 84) pour disposer un élément de commande (36) dans une position initiale avant une opération de préparation pour décharger l'air dans le récipient (16) ;
l'élément de commande (36) étant agencé de sorte que pendant l'opération de préparation, un mouvement de l'élément de commande (36) dans la direction axiale vers le trou de buse (20a) amène le cylindre interne (28) et le poussoir (34) à se déplacer dans la direction axiale vers le trou de buse (20a) ;
une partie de butée (38b) qui est agencée de sorte qu'elle arrête le mouvement de l'élément de commande (36) vers le trou de buse (20a) dans une position de butée prédéterminée ;
**caractérisé par** :
des moyens de retour comprenant un élément élastique qui est agencé de sorte qu'il pousse l'élément de commande (36) de la position de butée jusqu'à une position d'attente qui est entre la position de butée et la position initiale dans la direction axiale ;
dans lequel, après l'opération de préparation pendant l'utilisation du distributeur à volume constant (14), le poussoir (34) est déplacé par un mouvement de l'élément de commande (36) de la position d'attente à la position de butée, le poussoir (34) étant déplacé sur une plus courte distance que lors de l'opération de préparation.

2. Distributeur à volume constant (14) selon la revendication 1, dans lequel la partie de butée (38b) est prévue dans le cylindre externe (26).

3. Distributeur à volume constant (14a) selon la revendication 1, comprenant en outre un élément de couvercle prévu au niveau d'une extrémité sur le côté de trou de buse du cylindre externe (26) pour arrêter le cylindre interne (28a), dans lequel la partie de butée (52b) est prévue dans le cylindre interne (28a) qui est arrêté par l'élément de couvercle.

4. Distributeur à volume constant (14) selon l'une quelconque des revendications 1 à 3, comprenant en outre des moyens de restriction pour limiter le mouvement de l'élément de commande (36) disposé dans la position initiale par les moyens de disposition (44, 46, 84) de sorte que l'élément de commande (36) ne se déplace pas dans la direction axiale vers le trou de buse (20a).
